# EUROPEAN PATENT APPLICATION

(11) **EP 0 712 897 A1**
(43) Date of publication of application: **22.05.1996**
(21) Application number: 95116645.3
(22) Date of filing: 23.10.1995
(51) Int. Cl.: C08L 33/02, C08L 71/02, C08L 39/06, C11D 3/37, A61K 7/00

(54) **Thickening compositions**

(30) Priority: 17.11.1994 IT MI942329
(71) Applicant: 3V SIGMA S.p.A, I-20121 Milano (IT)
(72) Inventor: Berte, Ferruccio, I-24121 Bergamo (IT); Barzaghi, Massimo, I-24121 Bergamo (IT)
(74) Representative: Minoja, Fabrizio

(57) **Abstract**

Thickening compositions consisting of:
a) 45-98% by weight of polymers or copolymers, optionally cross-linked, of an unsaturated carboxylic acid constituting at least 70% by weight of the copolymer;
b) 55-2% by weight of polyvinylpyrrolidone and/or of polyglycols of formula (I) 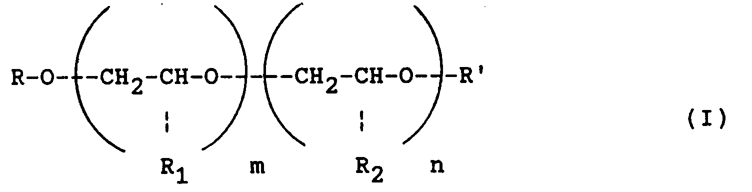
c) 0-5% by weight of an inorganic or sulfonic acid and/or the salts thereof;
wherein the compounds are as defined in the disclosure.

## Description

The present invention relates to thickening compositions consisting of:
a) 45-98% of polymers or copolymers, optionally cross-linked, of an unsaturated carboxylic acid;
b) 55-2% of polyvinylpyrrolidone and/or of a polyglycol of formula (I) 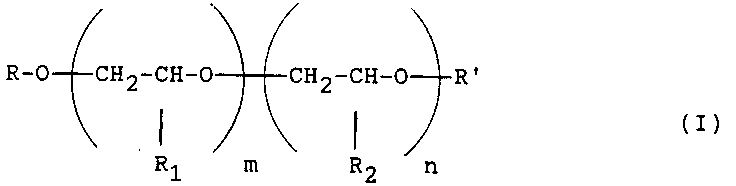
wherein R and R' can be the same or different and represent hydrogen, linear or branched C₁-C₈ alkyl or optionally substituted phenyl, R₁ and R₂ are the same or different and represent hydrogen or the methyl or ethyl group;
and the expression 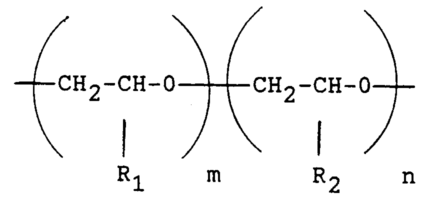
means that the units 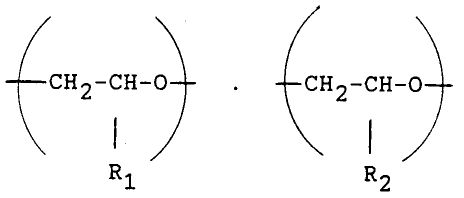
can be present in any order from random to blocks,
n and m can be the same or different and can have values comprised between 0 and 10,000 with the proviso that their sum is at least 5 and at most 10,000.

Up to 5% by weight of a component c) comprising an inorganic or sulfonic acid or their salts with alkali metals, alkaline-earth metals, aluminium, zinc, ammonia or amines may be optionally added to the compositions.

A further object of the invention are the aqueous dispersions obtained from the compositions described above and their use as thickening agents.

Polymers or copolymers of unsaturated carboxylic acid, such as acrylic, methacrylic, maleic, fumaric, itaconic, crotonic, sorbic acids and particularly those of methacrylic or acrylic acid cross-linked with vinyl ethers, specifically with polyallyl saccharose or polyallyl pentaerythritol or divinyl glycol, have been known for some time and extensive patent literature concerning them exists. These polymers, and mainly the acrylic acid cross-linked ones, are widely employed as highly effective thickeners.

The success attained by this class of thickeners in a number of industrial fields such as, the textile, cosmetic, pharmaceutical industries and in the formulation of detergents, is mainly due to the extraordinary thickening and suspending power, to rheology, to the lack of toxicity, to their resistance and consistency, which they impart, through their use, to the formulations.

They are generally marketed in the acidic form as a fine, soft powder. During use, the acid polymers, upon dispersion in water or in other solvents, followed by neutralization with bases, develop their thickening action, reaching very high levels of viscosity, even at very low dosage levels.

Problems which have not yet been resolved in the use of these polymers involve certain drawbacks during the application phase. In fact the polymers are fine, soft and very light powders which very easily disperse in the environment. Besides they tend to become electrostatically charged and adhere to many kinds of surfaces. This makes their handling not only very troublesome, but also sometimes difficult.

As mentioned above, to use said polymers, a homogeneous aqueous dispersion of its acidic form has to be prepared and subsequently neutralized. However these polymers, even though they are hygroscopic are not easy to wet, moreover they tend to form jelly blocks, partially swelled and very difficult to disintegrate, therefore time-consuming cautions should be taken in order to avoid their formation.

On the other hand, any concentrated dispersions (mothers) cannot be used from which various formulations could be prepared. In fact these aqueous dispersions have very high viscosities, which are unpractical, even at 2-3% concentrations.

In order to make the polymers more dispersible in water, modifications have been suggested, as described for example in US 5 288 814, but they were unsuccessful in practice.

Methods have been proposed for the preparation of concentrated aqueous dispersions, about 5% of acid polymer, by addition of small amounts of salts of acids with polyvalent cations, as described in WO 9300369, but they were practically unsuccessful in that the viscosities at such concentrations are still too high for a possible use, even after addition of the electrolyte.

On the other hand, the electrolyte amount cannot be increased in order to lower further the viscosity of the dispersions since, as it is well known, electrolytes cause a marked decrease in the thickening power of this class of polymers, with a serious loss of effectiveness.

The problem of the technological need to provide a thickening agent which is highly effective in a solid or fluid, preferably fluid, form as well as easy to handle and use, is still unresolved.

It has surprisingly been found that using the compositions according to the present invention, low-viscosity, fluid, concentrated aqueous dispersions of the carboxylic polymers can be prepared, which can subsequently be used easily. Using such ready-to-use concentrated dispersions, any thickened formulation can be prepared easily and in short time, whereas the use of the powdered polymer doesn't attain such a result.

In fact the polymer, being already pre-dispersed by dilution and subsequent neutralization with an alkali hydroxide, ammonia or an amine, depending on the type of formulation, immediately exerts its thickening power.

The polymeric thickening agents according to the present invention are polymers or copolymers, preferably cross-linked, of unsaturated carboxylic acids such as: acrylic, methacrylic, crotonic, sorbic, itaconic, β-acryloxypropionic, maleic, fumaric acids. In the case of copolymers, these contain at least 70% by weight of the above mentioned unsaturated acids. Possible comonomers are for example the esters of the above mentioned unsaturated carboxylic acids with aliphatic alcohols having 1 to 30 carbon atoms, with alcohols or polyethoxylated and/or polypropoxylated phenols; hydroxylalkyl acrylates or methacrylates; alkoxyalkyl acrylates and methacrylates; acrylonitrile, methacrylonitrile, acrylamide or methacrylamide and the N-substituted derivatives thereof; vinyl alcohol esters, vinyl ethers, ethylene, propylene, styrene and butadiene monomers.

The carboxylic polymers according to the present invention are preferably cross-linked with polyunsaturated compounds such as: divinylbenzene, allyl acrylates and methacrylates, glycol diacrylates and dimethylacrylates, 1,7-octadiene, butadiene, triallylcyanurate or isocyanurate, allylacrylamide and allylmethacrylamide and particularly polyallyl ethers of polyols such as polyallyl saccharose and polyallyl pentaerythritol and divinyl glycol.

The compositions according to the present invention have the advantage of being less pulverulent, more easily and quickly dispersible in water and making it possible to prepare concentrated, fluid aqueous dispersions of the polymeric thickening agents.

The polyglycols of formula (I) according to the invention are polymers or copolymers obtained from ethylene oxide and/or propylene oxide and/or butylene oxide, or they are the polycondensation products of the oxides cited above with aliphatic alcohols or optionally substituted phenols.

As component b), according to the invention, a polyvinylpyrrolidone can also be used.

With compositions containing only the carboxylic polymer and the component b), (polyglycol of formula (I) or polyvinylpyrrolidone), concentrated aqueous dispersions of the polymeric thickening agent are obtained which have a low viscosity and are already fluid enough to be used without difficulties.

Adding the component c), a synergistic effect is obtained, i.e. the aqueous dispersions are further fluidized, therefore even more concentrated and easy to handle fluid dispersions can be prepared.

The compounds constituting the component c) are inorganic acids, such as sulfuric, hydrochloric, phosphoric and nitric acids; or sulfonic acids such as paratoluenesulfonic acid, methanesulfonic acid or the corresponding salts thereof, for example with alkali or alkaline-earth metals, zinc, aluminium or with ammonia, amines or polyamines.

The component c) is added in an amount ranging from 0.001 to 5%, since in higher amounts it causes a drastic decrease in the thickening power of the carboxylic polymer.

The compositions according to the present invention can be prepared easily mixing homogeneously the carboxylic polymers with the components b) and optionally c). The mixing can be carried out immediately after the polymerization has been completed, adding to the slurry of the polycarboxilic acid, prepared for example, as described in US 2 798 053, a product constituting the component b) and optionally a compound constituting the component c), subsequently the solvent is removed and the residue is dried to obtain the composition according to the invention in form of a powder.

The compositions of the invention can also be prepared directly mixing homogeneously in a suitable mixer the carboxylic polymer already recovered in the form of a powder with the other two components or suspending the three components in a suitable solvent with stirring, and subsequently evaporating to dryness the mixture.

The present invention also comprises the fluid, concentrated aqueous dispersions, obtained using the compositions of the present invention.

The concentrated dispersions of the compositions of the invention can be prepared directly in situ, i.e. dispersing the carboxylic polymer, together with components b) and c), in any order in an aqueous medium at a temperature ranging from 2 to 100°C and then stirring until obtaining a fluid, concentrated, ready-to-use dispersion.

Alternatively, the dispersions can be prepared under the same conditions, dispersing directly the already formulated compositions.

The following examples illustrate the present invention.

### Example 1 - Preparation of the polyacrylic acid

98.4 g of acrylic acid, 1.10 g of pentaerythritol polyallyl ether and 0.50 g of di(tert-butylcyclohexyl)peroxidicarbonate in 1320 g of methylene chloride are refluxed for 10 hours.

From the final slurry, removing the solvent, 100 g of cross-linked polyacrylic acid in the form of a soft powder are obtained.

### Examples 2-6

Operating as described in Example 1, 497 g of a slurry of cross-linked polyacrylic acid, before removing the solvent, are added with various amounts of polyglycols of formula (I) to obtain the compositions listed in Table I.

**TABLE I**

| Example | polyglycol of formula I | Polyglycol g |
|---|---|---|
| 2 | polyethylene glycol 8,000 | 7.0 |
| 3 | polyethylene glycol 3,000 | 12.2 |
| 4 | polyethylene glycol 8,000 | 10.5 |
| 5 | polyethylene glycol 35,000 | 14.0 |
| 6 | copolymer (ethylene oxide/propylene oxide) 3,000 | 12.2 |

### Examples 7-13

Operating as described in Examples 2-6, 497 g of a slurry of cross-linked polyacrylic acid are added with, besides the polymers constituting the component b), various amounts of acids or salts, as reported in Table II.

**TABLE II**

| Example | Component b) | g | Salt or acid | g |
|---|---|---|---|---|
| 7 | polyethylene glycol 1500 | 7.0 | sodium sulfate | 0.24 |
| 8 | polyethylene glycol 8,000 | 7.0 | sodium sulfate | 0.04 |
| 9 | polyethylene glycol 8,000 | 7.0 | sodium sulfate | 0.35 |
| 10 | polyethylene glycol 8,000 | 7.0 | methanesulfonic acid | 0.04 |
| 11 | polyethylene glycol 8,000 | 7.0 | magnesium sulfate heptahydrate | 0.04 |
| 12 | eo/po copol. 3,000 | 7.0 | sodium sulfate | 0.12 |
| 13 | PVP K-15 | 14.0 | sodium sulfate | 0.18 |
| wherein PVP K-15 is polyvinylpyrrolidone 10,000 | | | | |

### Example 14

30.0 g of powdery cross-linked polyacrylic acid, obtained as in Example 1, are mixed with 6.0 g of polyethylene glycol 8,000 in a suitable mixer until obtaining a homogeneous composition in the form of a powder.

### Example 15

30.0 g of powdery cross-linked polyacrylic acid, obtained as in Example 1, are mixed with 6.0 g of finely subdivided polyethylene glycol and 0.30 g of sodium sulfate, to obtain a homogeneous composition in the form of a powder.

### Aqueous dispersions

The compositions obtained in Example 1-15 are dispersed with stirring in demineralized water at room temperature, to obtain in a short time fluid, homogeneous aqueous concentrates of cross-linked polyacrylic acid.

Table III reports the concentrations referred to polyacrylic acid, the viscosities of the dispersions and the viscosities of 0.5% gels by weight of active substance obtained by dilution and neutralization of said dispersions with sodium hydroxide. Viscosities are measured with a Brookfield RVT viscometer at 25°C and 20 rpm.

**TABLE III**

| Composition Example | Concentration % weight | Dispersion viscosity mPa.s | Gel viscosity mPa.s |
|---|---|---|---|
| 1 no addition | 6 | 90,000 | 52,000 |
| 2 | 6 | 15,000 | 52,000 |
| 3 | 7 | 1,700 | 50,500 |
| 4 | 8 | 3,000 | 51,000 |
| 5 | 7 | 3,800 | 50,500 |
| 6 | 7 | 3,850 | 52,000 |
| 7 | 6 | 12,000 | 50,000 |
| 8 | 6 | 5,300 | 50,500 |
| 9 | 6 | 1,700 | 53,000 |
| 10 | 6 | 1,000 | 50,000 |
| 11 | 6 | 2,500 | 50,500 |
| 12 | 6 | 6,000 | 50,500 |
| 13 | 6 | 4,000 | 51,000 |
| 14 | 6 | 15,000 | 50,500 |
| 15 | 6 | 2,000 | 51,000 |

### Example 16 - Comparison with WO 9300369

18 g of polyacrylic acid of Example 1 are dispersed with stirring in a solution of 0.36 g of magnesium sulfate heptahydrate in 281.6 g of demineralized water, stirring to a homogeneous dispersion.

### Example 17 - Comparison with WO 9300369

The procedure described in Example 16 is followed, but adding 1.8 g of magnesium sulfate heptahydrate.

### Example 18

The procedure described in Example 16 is followed, but adding 5.4 g of polyethylene glycol 8,000 instead of magnesium sulfate heptahydrate.

### Example 19

The procedure of Example 16 is repeated, also adding 0.08 g of magnesium sulfate heptahydrate together with 3.6 g of polyglycol 8,000.

Table IV reports the viscosity of the dispersions of Examples 15-18 and the viscosity of the gels with 0.5% of active substance obtained therefrom.

**TABLE IV**

| Example | Dispersion viscosity mPa.s | Gel viscosity mPa.s |
|---|---|---|
| 16 | 44,000 | 50,000 |
| 17 | 30,000 | 34,000 |
| 18 | 3,500 | 52,000 |
| 19 | 1,700 | 50,000 |

### Example 20

24.0 g of Carbopol 980 (a cross-linked polyacrylic acid produced by B.F. Goodrich - USA) are dispersed with stirring in a solution of 4.8 g of polyethylene glycol 6,000 in 271.2 g of demineralized water. A homogeneous concentrated dispersion is obtained, with viscosity of 6,500 mPa.s.

### Example 21

18.0 g of Stabilen 30 (an acrylic acid cross-linked copolymer produced by 3V SIGMA - Italy) are dispersed in a solution of 3.6 g of polyethylene glycol 8,000 in 278.4 g of demineralized water, yielding a homogeneous concentrated dispersion with viscosity 14,000 mPa.s.

### Example 22

30.0 g of Polygel DB (a cross-linked polyacrylic acid produced by 3V SIGMA - Italy) are dispersed in a solution of 6.0 g of polyethylene glycol 6,000 and 0.10 g of sulfuric acid in 263.9 g of demineralized water to obtain a concentrated dispersion with viscosity 4,000 mPa.s.

## Claims

1. Thickening compositions consisting of:
a) 45-98% by weight of optionally cross-linked polymers or copolymers of an unsaturated carboxylic acid which constitutes at least 70% by weight of the copolymer;
b) 55-2% by weight of polyvinylpyrrolidone and/or of polyglycols of formula (I) 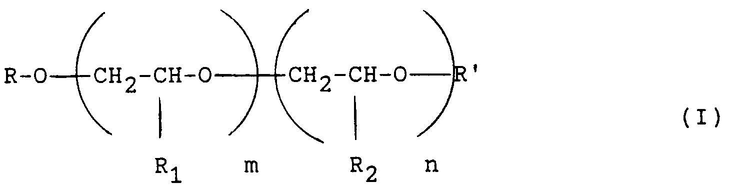
wherein R and R' are the same or different and represent hydrogen, straight or branched C₁-C₈ alkyl or optionally substituted phenyl,
R₁ and R₂ are the same or different and represent hydrogen or the methyl or ethyl group;
the expression 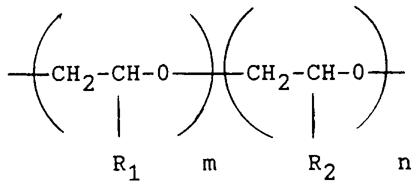 means that the units 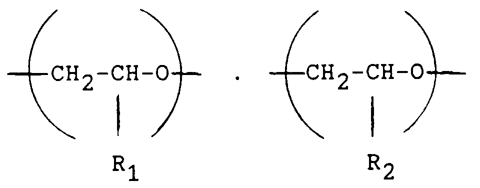 can be present in any order from random to blocks,
n and m can be the same or different and can have values comprised between 0 and 10,000, with the proviso that m+n ranges from 5 to 10,000.

2. Compositions according to claim 1 consisting of:
a) 45-98% by weight of cross-linked polymers or copolymers consisting by at least 70% by weight of acrylic and/or methacrylic acids.
b) 55-2% by weight of polyvinylpyrrolidone and/or of polyglycol of formula (I).

3. Compositions according to claim 1 consisting of:
a) 60-95% by weight of cross-linked polymers or copolymers consisting by at least 70% by weight of acrylic and/or methacrylic acids.
b) 40-5% by weight of polyvinylpyrrolidone and/or of polyglycol of formula (I).

4. Compositions according to claims 1-3 containing up to 5% by weight of a sulfonic or inorganic acid or of a salt thereof.

5. Compositions according to claims 1-4 containing up to 2% of methanesulfonic, toluenesulfonic, xylenesulfonic, hydrochloric, sulfuric, nitric or phosphoric acids or a salt thereof with alkali or alkaline-earth metals, aluminium, zinc or with ammonia, amines or polyamines.

6. Compositions according to claims 1-3, wherein the component b) is a polyglycol of formula I.

7. Compositions according to claim 6, wherein the component b) is a polyethylene glycol of molecular weight from 1,000 to 100,000.

8. Compositions according to claims 1-3, wherein the component b) is a copolymer ethylene oxide/propylene oxide with weight molecular from 1,000 to 100,000.

9. Compositions according to claims 1-3, wherein the component b) is polyvinylpyrrolidone.

10. Aqueous dispersions containing 3 to 40% by weight of the compositions according to claims 1-9.

11. Aqueous dispersions containing 5 to 30% by weight of the compositions according to claims 1-9.

12. A process for the preparation of the compositions according to claims 1-9 in the form of a powder or granulate, wherein the component b) and optionally the acids or salts thereof according to claims 4 and 5, are mixed with the component a), when the polymerization has been completed or subsequently, with stirring and in the presence of a suitable solvent which is finally removed by drying.

13. A process for the preparation of the compositions according to claims 1-9 in the form of a powder or granulate, wherein the components constituting the compositions are mixed in a suitable mixer.

14. A process for the preparation of the aqueous dispersions according to claims 10-11, wherein the compositions according to claims 1-9 are dispersed in the aqueous medium with stirring and at temperatures from 2 to 100°C.

15. A process for the preparation of the aqueous dispersions according to claims 10-11, wherein the components of the compositions according to claims 1-9 are dispersed in the aqueous medium in any order with stirring and at temperatures from 2 to 100°C.

16. The use of the compositions according to claims 1-9 as thickening agents.

17. The use of the aqueous dispersions according to claims 10-11 as thickening agents.
